# EUROPÄISCHE PATENTANMELDUNG

(11) **EP 4 488 375 A1**
(43) Veröffentlichungstag der Anmeldung: **08.01.2025**
(21) Anmeldenummer: 23184206.3
(22) Anmeldetag: 07.07.2023
(51) Int. Cl.: C12P 19/02, C12N 9/90

(54) **VERFAHREN ZUR HERSTELLUNG EINER WÄSSRIGEN LÖSUNG ENTHALTEND ALLOSE**

(71) Anmelder: Annikki GmbH, 8074 Raaba-Grambach (AT)
(72) Erfinder: Staunig, Nicole, 8074 Raaba-Grambach (AT)
(74) Vertreter: Schwarz & Partner Patentanwälte GmbH

(57) **Zusammenfassung**

Verfahren zur Herstellung einer wässerigen Lösung enthaltend Allose, indem aus D-Fructose, welche in einer wässerigen Lösung gelöst vorliegt, durch Behandeln mit einer Epimerase *in vitro* D-Psicose gebildet wird, wonach die D-Psicose durch Behandeln mit einer NAD(P)H-abhängigen Oxidoreduktase *in vitro* zu Allitol reduziert wird, wonach die Epimerase und die NAD(P)H-abhängige Oxidoreduktase deaktiviert oder durch Ultrafiltration entfernt werden und anschließend das Allitol enzymatisch in vitro zu Allose oxidiert wird.

## Beschreibung

Die vorliegende Erfindung betrifft ein Verfahren zur Herstellung einer wässrigen Lösung des seltenen Monosaccharids Allose.

### Hintergrund der Erfindung

### D-Allose

Die Aldohexose D-Allose ist das C3-Epimer der D-Glucose und aufgrund ihres geringen natürlichen Vorkommens ein sogenannter seltener Zucker. Sie ist beispielsweise in bestimmten Seegräsern (Kannan et al., 2012) oder als Bestandteil von Glykosiden in der in Südafrika beheimateten Pflanze *Protea rubropilosa* (Zuckerbusch) (Perold et al., 1973) zu finden.

D-Allose ist ein süß-schmeckender Zucker (80% Süße im Vergleich zu Saccharose), weist aber im Gegensatz zu dieser kaum Kalorien auf (Mooradian et al., 2017). Darüber hinaus hat D-Allose noch eine Fülle an weiteren positiven Eigenschaften wie eine antitumorale (z.B. bei Blasenkrebs (Tohi et al., 2022)), antioxidative (Ishihara et al., 2011) oder entzündungshemmende (Gao et al., 2011) Wirkung. Weitere Beispiele sowie weitere physiologische Effekt von D-Allose sind in Übersichtsartikeln angeführt (Chen et al., 2018; Lim & Oh, 2011; Mijailovic et al., 2021).

Da aufgrund der vielfältigen Anwendungsmöglichkeiten der Bedarf an D-Allose nicht über die natürlichen Vorkommen abgedeckt werden kann, wurden und werden synthetische Methoden (chemisch oder biotechnologisch) zur Herstellung von D-Allose ausgehend von häufiger vorkommenden (und damit günstigeren) Monosacchariden wie D-Fructose oder D-Glucose entwickelt.

US 9109266 B2 beschreibt die Umwandlung von D-Fructose zu D-Allose mittels NaOH oder stark basischen lonentauscherharzen (nach dem Mechanismus der Lobry-de-Bruyn-Alberda-van-Ekenstein-Umlagerung), was allerdings zu einer Vielzahl an Nebenprodukten wie D-Glucose, D-Mannose, D-Altrose sowie D-Psicose führt, die abgetrennt werden müssen.

US 5433793 offenbart die von Ammoniummolybdat katalysierte Isomerisierung von D-Glucose zu D-Allose, die bei 130 °C in einem essigsauren wässrigen Medium durchgeführt wird. Damit können in etwa 10 Gewichtsprozent D-Allose (bezogen auf die Trockenmasse) erhalten werden, allerdings erfordert die Isolierung des Produkts Aktivkohle-Filtration, lonenaustausch- und SMB- (Simulated Moving Bed)- Chromatographie.

Jumde et al. (2016) präsentierten eine chemische Syntheseroute ausgehend von D-Glucose, die eine Palladium-katalysierte regioselektive Oxidation an der C3-Position sowie eine stereoselektive Reduktion zu D-Allose mittels Borhydrid-basierten Reagenzien beinhaltet.

WO 1997/042339 A1 beschreibt ein Verfahren zur Umwandlung von Saccharose zu D-Allose und zum entsprechenden Zuckeralkohol Allitol. Durch Fermentation mit *Agrobacterium tumefaciens* wird Saccharose zu 3-Ketosaccharose oxidiert, die durch Hydrierung (an Raney-Nickel) zu Allosaccharose umgesetzt wird. Die Spaltung der Allosaccharose kann säurekatalysiert (Kationentauscher) oder enzymatisch durch Invertase oder β-Fructosidase durchgeführt werden. Die Trennung der D-Allose/D-Fructose-Mischung kann entweder durch Entfernung der D-Fructose mittels Hefe-Fermentation oder durch chromatographische Methoden (lonenaustauscher) erfolgen. Allitol kann durch Hydrierung der D-Allose (an Raney-Nickel) hergestellt werden.

Chemische Verfahren wie die oben genannten zeichnen sich im Allgemeinen durch niedrige Umsätze, komplexe Reaktionsschritte mit schlechter Atomökonomie und/oder Bildung von Nebenprodukten aus und sind daher für die Herstellung von D-Allose im großtechnischen Maßstab gänzlich ungeeignet.

Die ineffizienten chemischen Syntheserouten wurden mittlerweile von effizienteren biotechnologischen Verfahren verdrängt.

Von D-Glucose ausgehend führen drei enzymatische Schritte zur D-Allose. D-Glucose wird zuerst mit einer Glucose/Xylose-Isomerase (GI/XI) zu D-Fructose isomerisiert (Nam, 2022), welche mit einer 3-Ketose-Epimerase (D-Tagatose-3-epimerase (DTE), D-Psicose/Allulose-3-epimerase (DPE/DAE) oder L-Ribulose-3-epimerase (LRE)) zu D-Psicose, auch bekannt als D-Allulose, epimerisiert wird (Zhang et al., 2016; Jiang et al., 2020). Der abschließende Schritt (D-Psicose → D-Allose) wird durch die Einwirkung verschiedener Isomerasen - L-Rhamnose-Isomerase (L-RI, EC 5.3.1.14), D-Ribose-5-phosphat-Isomerase (RPI, EC 5.3.1.6), D-Galactose-6-phosphat-lsomerase (GaPI, EC 5.3.1.26) sowie D-Glucose-6-phosphat-Isomerase (GlPI, EC 5.3.1.9) - bewerkstelligt (Chen et al., 2018; Lim & Oh, 2011).

L-Rhamnose-Isomerasen, die mit Abstand wichtigste Enzymklasse zur Isomerisierung von D-Psicose und D-Allose (Chen et al., 2018), sind in beispielsweise in EP 1589102 B1, EP 1788089 B1, EP 1860195 B1, US 7205141 B2, US 7501267 B2, US 7691619 B2 sowie US 8748589 B2 beschrieben.

Die Umsetzung von D-Fructose zu D-Psicose mittels Ketose-3-Epimerase läuft nicht vollständig ab, vielmehr bildet sich ein Gleichgewichtsverhältnis zwischen beiden Epimeren aus. Je nach Reaktionsbedingungen (Temperatur zwischen 40 und 70 °C, pH-Wert zwischen 6 und 11) liegt dieses zwischen 80:20 und 62,5:37,5 (D-Fructose : D-Psicose) (Zhang et al., 2016; Jiang et al., 2020).

Auch bei der nachfolgenden Isomerisierung handelt es sich um eine Gleichgewichtsreaktion, bei der das Gleichgewicht aufseiten der D-Psicose liegt - je nach Bedingung liegt das Massenverhältnis zwischen 77:23 und 62,5:37,5 (D-Psicose : D-Allose) (Chen et al., 2018; Lim & Oh, 2011).

Lee et al. (2018) beschrieben ein Verfahren zur Umwandlung von D-Fructose zu D-Allose mittels DPE aus *Flavonifractor plautii* and RPI (R132E-Mutante) aus *Clostridium thermocellum.* 600 g/l D-Fructose konnten innerhalb von 2 h zu 79 g/l D-Allose umgesetzt werden, was einem Umsatz von 13% entspricht. Zusätzlich werden 1 mM Co²⁺-Ionen als Kofaktor für die DPE benötigt.

Li et al. (2020) präsentierten ein ähnliches Verfahren - DPE aus *Ruminococcus sp.* und L-RI aus *Bacillus subtilis* wurden in *Escherichia coli* exprimiert und auf Harzen immobilisiert. Nach 5 h Reaktionszeit wurde ein Gleichgewicht mit einem Massenverhältnis von 66:24:10 (D-Fructose : D-Psicose : D-Allose) erreicht (bei 500 g/l D-Fructose als Substrat).

In US 10689668 B2 werden Fusionsenzyme bestehend aus einer DPE aus *Ensifer adhaerens* oder *Clostridium scindens* sowie einer RPI aus *Persephonella marina* EX-H1 beschrieben (EDPE_RPI oder CDPE_RPI), mit denen auch Umsätze von bis zu 13% erreicht werden können.

Eine Alternative zu den oben genannten Isomerasen ist eine kommerzielle Glucose-Isomerase von Sweetzyme IT, die von Choi et al. verwendet wurde, um D-Psicose zu D-Allose zu isomerisieren. Das Enzym wurde in einem Festbett-Reaktor immobilisiert, was eine einfache Abtrennung der Produktlösung erlaubt (Choi et al., 2021).

Da durch die Kombination der Epimerisierung und Isomerisierung kein vollständiger Umsatz erreicht werden kann, muss D-Allose aus den entstehenden Produktgemischen abgetrennt werden.

Je nach verwendeter Isomerase kommt zum Teil noch erschwerend hinzu, dass aus D-Psicose auch D-Altrose, das C2-Epimer der D-Allose, entstehen kann (Chen et al., 2018; Lim & Oh, 2011). Durch Verwendung einer rekombinanten L-RI aus *Pseudomonas stutzeri* LL172 beispielsweise wurde ausgehend von D-Psicose eine Produktmischung bestehend aus 25% D-Allose, 8°/ D-Altrose und 67% D-Psicose erhalten (Menavuvu et al., 2006).

Für die Isolierung der D-Allose aus enzymatischen Umsätzen bieten sich zwei Methoden an: 1) Kristallisation aus einer aufkonzentrierten Zuckerlösung durch Zugabe von Ethanol (Menavuvu et al., 2006) oder 2) SMB-Chromatographie mit anschließender Kristallisation aus dem Sirup (Morimoto et al., 2006).

Des Weiteren sind auch fermentative Verfahren zur Synthese von D-Allose bekannt. Zheng et al. (2022) konstruierten einen neuen Stoffwechselpfad in *E. coli* basierend auf Gl, DAE und RPI, um D-Glucose in D-Allose umzuwandeln - 0,127 g/l D-Allose konnten auf diese Weise nach 84 h erhalten werden, was einer Ausbeute von 0,045 g D-Allose pro g D-Glucose entspricht.

Eine Möglichkeit zur Umgehung der thermodynamisch ungünstigen Epimerisierungs- und Isomerisierungsreaktionen sind Enzym-Kaskaden mit phosphorylierten Intermediaten. Der letzte Schritt, die Dephosphorylierung, ist irreversibel und treibt somit die Kaskade an (Li et al., 2021).

Eine Kaskade, die in US 10745683 B2 und US 11236320 B2 beschrieben wird, weist D-Glucose-1-phosphat (G1P) als zentrales Intermediat auf. G1P wird mittels Phosphoglucomutase zuerst zu D-Glucose-6-phosphat (G6P) und dann weiter mittels Phosphoglucose-Isomerase zu D-Frucose-6-phosphat (F6P) umgewandelt. F6P wird danach mittels D-Psicose-6-phosphat-3-Epimerase zu D-Psicose-6-phosphat epimerisiert, welches anschließend mit D-Allose-6-phosphat-Isomerase zu D-Allose-6-phosphat umgesetzt wird. Der abschließende Schritt ist die Dephosphorylierung zu D-Allose mit D-Allose-6-phosphat-Phosphatase.

G1P kann beispielsweise durch die Einwirkung von Phosphorylasen auf z.B. Amylodextrin (erhalten durch die Hydrolyse von Stärke) oder Saccharose unter Verbrauch von Phosphat direkt hergestellt werden. Da die endständigen Zuckermonomere der Oligo- und Polysaccharide von den entsprechenden Phosphorylasen nicht phosphoryliert werden können, muss auf die Polyphosphat-Glucokinase (D-Glucose → G6P) bzw. Polyphosphat-Fructokinase (D-Fructose --> F6P) zurückgegriffen werden, wobei noch zusätzlich Polyphosphate als Phosphat-Quelle zugesetzt werden müssen, um die Ausbeuten zu steigern (US 10745683 B2; US 11236320 B2).

Ein großer Nachteil der Route, welche über phosphorylierte Zucker-Derivate abläuft, ist der Einsatz von teuren, energiereichen Phosphat-Verbindungen wie Polyphosphat in stöchiometrischen Mengen zur Einführung der Phosphat-Gruppen. Durch die Verwendung von Phosphorylasen kann dieses Problem zum Teil umgangen werden, jedoch können endständige Monosaccharide nicht ohne Zuhilfenahme von energiereichen Phosphat-Verbindungen phosphoryliert werden. Außerdem müssen die verbliebenen Phosphat-Verbindungen und Phosphat-Ionen nach Beendigung der Reaktion entfernt werden.

### L-Allose

Im Vergleich zu ihrem Enantiomer D-Allose kommt L-Allose natürlich nicht vor. Es sind nur wenige Verfahren zur Herstellung von L-Allose bekannt. Eine biotechnologische Route wurde von Terami et al. vorgestellt, welche vom ebenfalls seltenen Monosaccharid L-Psicose ausgeht. Dieses wird mittels einer immobilisierten L-Ribose-Isomerase aus *Cellulomonas parahominis* MB426 zu L-Allose isomerisiert. Im Gleichgewicht liegt eine Mischung von 33:77 (L-Allose : L-Psicose) vor, welche chromatographisch getrennt wird (Terami et al., 2015).

### Allitol als Intermediat

Durch die Reduktion an der C1-Position der D-Allose kann der achirale Zuckeralkohol Allitol erhalten werden (siehe z.B. WO 1997/042339 A1), der als Schnittstelle zwischen den D- und L-Hexosen eine zentrale Rolle in der sogenannten Izumoring-Strategie zur Bioproduktion von seltenen Zuckern einnimmt (Izumori, 2006; Hassanin et al., 2017).

Allitol wiederum kann ausgehend von D-Fructose und einer Kombination bestehend aus DTE, Ribitol-Dehydrogenase (RDH) und Formiat-Dehydrogenase (FDH; als Regenerationsenzym für den Kofaktor Nicotinamidadenindinukleotid (NADH)) hergestellt werden. Auf diese Weise kann das Gleichgewicht vollständig zur Produktseite (D-Psicose und in weiterer Folge Allitol) verschoben und Allitol in hoher Reinheit gewonnen werden (Takeshita et al., 2000).

Wang et al. (2023) beschreiben einen *E*. *coli*-Ganzzell-Biokatalysator zur Umwandlung von D-Fructose zu Allitol. Die verwendeten *E. coli*-Zellen beinhalteten eine DPE aus *Clostridiales,* eine RDH aus *Providencia alcalifaciens*, eine FDH aus *Starkeya* sowie eine weitere DPE aus *Rhizobium straminoryzae.* Auf diese Weise wurde D-Fructose (500 mM = 90 g/l) innerhalb von 12 h bei 37 °C und pH 6 unter Einsatz von 1000 mM Natrium-Formiat zu 452 mM Allitol umwandelt (Umsatz 90,4%). Als Nebenprodukt entstand ca. 30 mM D-Sorbitol (das Reduktionsprodukt der D-Fructose).

Durch anschließende Oxidation der Hydroxy-Gruppe an der C1-Position von Allitol gelangt man zu D-Allose und an der C6-Position zu L-Allose. Für beide Oxidationen sind zum jetzigen Zeitpunkt keine biotechnologischen Verfahren bekannt.

Basierend auf diesen Erkenntnissen setzt sich die vorliegende Erfindung zur Aufgabe, ein effizientes enzymatisches Verfahren zur Umsetzung von D-Fructose zu Allose zur Verfügung zu stellen.

### Detaillierte Beschreibung der Erfindung

Diese Aufgabe wird gelöst, indem bei einem Verfahren zur Herstellung einer wässerigen Lösung enthaltend Allose, indem aus D-Fructose, welche in einer wässerigen Lösung gelöst vorliegt, durch Behandeln mit einer Epimerase *in vitro* D-Psicose gebildet wird, wonach die D-Psicose durch Behandeln mit einer NAD(P)H-abhängigen Oxidoreduktase in vitro zu Allitol reduziert wird, wonach die Epimerase und die NAD(P)H-abhängige Oxidoreduktase deaktiviert oder durch Ultrafiltration entfernt werden und anschließend das Allitol enzymatisch *in vitro* zu Allose oxidiert wird.

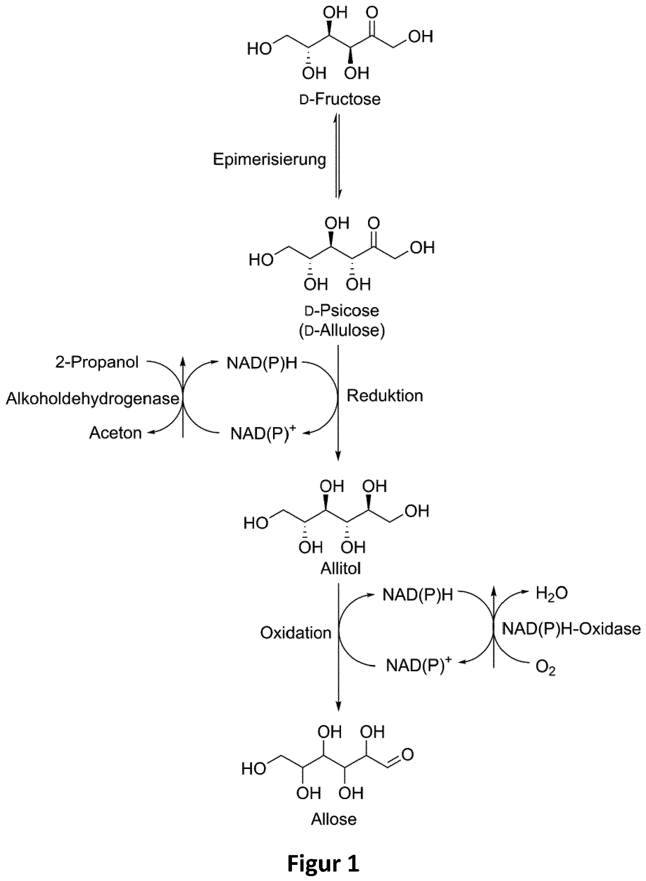

Die Reaktionsschritte des erfindungsgemäßen Verfahrens sind in der beiliegenden Figur 1 schematisch dargestellt.

Bei einer bevorzugten Variante des erfindungsgemäßen Verfahrens wird der durch die Reduktion entstandene oxidierte Kofaktor NAD(P)⁺ mittels einer Alkoholdehydrogenase und einem sekundären Alkohol unter Bildung eines Ketons reduziert.

Es hat sich gezeigt, dass sich als der sekundäre Alkohol 2-Propanol besonders eignet.

Ferner ist bevorzugt, wenn die enzymatische Oxidation des Allitols zu Allose mit einer entsprechenden NAD(P)⁺-abhängigen Oxidoreduktase durchgeführt wird.

Es hat sich gezeigt, dass das erfindungsgemäße Verfahren als Eintopf-Reaktion ohne Isolierung von Zwischenprodukten durchgeführt werden kann.

Die Erfindung betrifft ferner ein Verfahren zur Herstellung einer wässerigen Lösung enthaltend Allose, indem Allitol oxidiert wird, welches Verfahren dadurch gekennzeichnet ist, dass die Oxidation enzymatisch *in vitro* durchgeführt wird.

Die enzymatische Oxidation des Allitols zu Allose kann sehr gut mit einer entsprechenden NAD(P)⁺-abhängigen Oxidoreduktase durchgeführt werden.

Es hat sich ferner gezeigt, dass die Enzyme im erfindungsgemäßen Verfahren in einer Suspension, im Homogenat und/oder im Lysat der entsprechenden, sie bildenden Zellen vorliegen, wobei Lysate besonders bevorzugt sind.

Suspension bedeutet in diesem Kontext eine Suspension von *resting cells.* Diese werden nach der Kultivierung geerntet (vom Nährmedium abgetrennt) und in einem geeigneten Puffersystem suspendiert. Im Gegensatz zu fermentativen Verfahren, bei denen auch mit ganzen Zellen gearbeitet wird, können die *resting cells* aufgrund der Entfernung von Kohlenstoff-Quellen und Nährstoffen nicht mehr wachsen, sondern dienen nur der Umsetzung von Substraten (Lin & Tao, 2017). Homogenat steht in diesem Kontext für eine physikalisch und/oder chemisch behandelte Suspension (z.B. mittels Druckes, Lysozym oder Ultraschall behandelt), wobei die Zellbestandteile aus den Zellen freigesetzt werden. Ein Lysat wird erhalten, wenn die unlöslichen Zellbestandteile des Homogenats beispielsweise durch Filtration oder Zentrifugation entfernt werden (siehe *Produktion der Enzyme und Herstellung der Lysate* für Details).

In einer weiteren Variante können die Enzyme auch auf einem festen Trägermaterial immobilisiert sein.

Der durch die Oxidation entstandene, reduzierte Kofaktor NAD(P)H kann vorteilhaft mittels einer Oxidase und Sauerstoff zu NAD(P)⁺ oxidiert werden.

Vor der Ausführung des letzten Schritts (Oxidation) werden die Enzyme (Epimerase und Dehydrogenase) des ersten Schritts (D-Fructose → Allitol) an besten durch Hitzeeinwirkung deaktiviert oder durch Ultrafiltration entfernt, um die Bildung von Nebenprodukten durch die Enzyme zu verhindern.

Die besonders bevorzugte Konzentration von D-Fructose beträgt 50 - 250 g/l.

Der besonders bevorzugte Temperaturbereich für den ersten Schritt (Epimerisierung und Reduktion) liegt zwischen 25 und 45 °C, für den zweiten Schritt (Oxidation) zwischen 24 und 30 °C.

Der besonders bevorzugte pH-Bereich beider Schritte liegt zwischen 7 und 8,5.

In einer weiteren, besonders bevorzugten Ausführung des Verfahrens werden für die Konversion des Startmaterials nur Enzyme aus den Enzymgruppen Epimerasen, Dehydrogenasen, Reduktasen und Oxidasen verwendet, wobei eines oder mehrere dieser Enzyme aus jeder dieser Gruppen ausgewählt werden.

Die im Verfahren verwendete Epimerase kann aus einer der Gruppen EC 5.1.3.30 (D-Psicose-3-Epimerase) oder EC 5.1.3.31 (D-Tagatose-3-Epimerase/L-Ribulose-3-Epimerase) stammen, wobei die erstgenannte besonders bevorzugt ist.

Das zur Reduktion von D-Psicose verwendete Enzym stammt aus der Gruppe der Oxidoreduktasen, wobei eine Ribitol-Dehydrogenase (EC 1.1.1.56) besonders bevorzugt ist.

Das zur Oxidation von Allitol zu Allose verwendete Enzym stammt aus der Gruppe der Oxidoreduktasen, wobei eine Xylose-Reduktase oder Aldo/Keto-Reduktase besonders bevorzugt sind.

Die erfindungsgemäß eingesetzte Oxidoreduktase zur Oxidation von Allitol zu Allose ist vorzugsweise eine Xylose-Reduktase. Es hat sich überraschenderweise gezeigt, dass Xylose-Reduktasen in der Lage sind, D-Glucose in Anwesenheit des Kofaktors NAD(P)⁺ zu D-Sorbitol umzusetzen. Dementsprechend betrifft ein weiterer Aspekt der vorliegenden Erfindung die Verwendung von Xylose-Reduktasen zur Reduktion von Allitol zu Allose in Anwesenheit des Kofaktors NAD(P)⁺.

Die Oxidoreduktase zur Oxidation von Allitol zu Allose umfasst vorzugsweise eine Aminosäuresequenz, welche ausgewählt ist aus der Gruppe bestehend aus:
i) einer Aminosäuresequenz, die eine Identität SEQ ID Nr. 1, SEQ ID Nr. 3, SEQ ID Nr. 5, SEQ ID Nr. 7, SEQ ID Nr. 9, SEQ ID Nr. 11, SEQ ID Nr. 13, SEQ ID Nr. 15 oder SEQ ID Nr. 17 von mindestens 75% aufweist,
ii) einer Aminosäuresequenz, die durch eine Nukleinsäure kodiert wird, die eine Identität zu SEQ ID Nr. 2, SEQ ID Nr. 4, SEQ ID Nr. 6, SEQ ID Nr. 8, SEQ ID Nr. 10, SEQ ID Nr. 12, SEQ ID Nr. 14, SEQ ID Nr. 16 oder SEQ ID Nr. 18 von mindestens 75% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 2, SEQ ID Nr. 4, SEQ ID Nr. 6, SEQ ID Nr. 8, SEQ ID Nr. 10, SEQ ID Nr. 12, SEQ ID Nr. 14, SEQ ID Nr. 16 oder SEQ ID Nr. 18 bindet, oder besteht aus dieser, wobei stringente Bedingungen einen oder mehrere Waschschritte bei 65 °C und einer Salzkonzentration von 0,1x bis 2x SSC umfassen.

### SEQ ID Nr. 1:

MAPQIPNIKLSSGYDMPQVGFGLWKVDRSICADVVYNAIKIGYRLFDGACDYGNEVEAGQGIARAIKEGIVKR
EELFIVSKLWNTFHDGDKVEPIVRKQLADWGIDYFDLYLVHFPVALEYVDPSVRYPPGWFYDGEKEIRPSKATI
QETWTAMESLVEKGLARSIGVSNFQAQLLYDLLRYAKIRPATLQIEHHPFLVQQELLNLAKAEGIAVTAYSSFG
PQSFLEFNMKHAVQLTPLFEDETIKKIAAKYNRPASQVLLRWATQRGLAIIPKSTRPEIMKSNLESIEFDLSEEDI
ATISAFDRGLRFNQPTNYFPTEHLWIFG

### SEQ ID Nr. 2:

ATGGCTCCCCAGATCCCCAACATTAAGCTCAGCAGCGGCTATGACATGCCCCAGGTGGGCTTTGGACTGT
GGAAGGTCGACCGCTCGATCTGCGCCGACGTCGTCTACAACGCAATTAAGATTGGCTACCGCCTGTTTGA
CGGCGCTTGCGACTATGGCAACGAAGTCGAGGCCGGCCAGGGCATTGCCCGCGCCATCAAGGAGGGCA
TCGTGAAGCGTGAGGAGCTCTTCATCGTGTCCAAGCTCTGGAACACCTTCCACGACGGCGACAAGGTCG
AGCCCATCGTCCGCAAGCAGCTCGCCGACTGGGGCATTGACTACTTCGACCTCTACCTCGTCCACTTCCCT
GTCGCCCTCGAGTACGTCGACCCCTCGGTCCGCTACCCGCCCGGCTGGTTCTACGATGGCGAGAAGGAG
ATCCGCCCCAGCAAGGCCACCATCCAGGAGACCTGGACCGCCATGGAGTCGCTCGTTGAGAAGGGTCTG
GCCCGCAGCATTGGTGTCTCCAACTTCCAGGCCCAGCTCCTCTACGACCTGCTGCGCTACGCCAAGATCC
GCCCGGCCACCCTTCAGATCGAGCATCACCCCTTCCTCGTCCAGCAGGAGCTGCTCAACCTGGCCAAGGC
CGAGGGCATTGCCGTGACTGCCTACAGCTCATTCGGCCCTCAGAGCTTCCTCGAGTTCAACATGAAGCAC
GCCGTGCAGCTCACCCCGCTCTTCGAGGACGAGACCATCAAGAAGATCGCCGCCAAGTACAACCGTCCTG
CTTCGCAGGTTCTCCTGCGCTGGGCCACTCAGCGCGGTCTGGCCATTATCCCCAAGAGCACGCGCCCCGA
GATCATGAAGTCCAACCTCGAGAGCATCGAGTTCGACCTCAGCGAGGAAGATATTGCCACCATCTCGGCC

TTCGACCGCGGCCTGCGCTTCAACCAGCCCACAAACTACTTCCCCACCGAGCACCTCTGGATCTTTGGCTA
A

### SEQ ID Nr. 3:

MATPTIKLNSGYDMPLVGFGLWKVNKETCADQVYEAIKAGYRLFDGACDYGNEVEAGQGVARAIKEGIVKR
EDLFIVSKLWNTFHEADKVEPIARKQLADWGLDYFDLYLIHFPIALKYVDPAEIYPPGWTGTKKEVEFSNATIQE
TWQAMETLVDKKLTRSIGISNFSAQLIMDLLRYARIRPATLQIEHHPYLTQQALVEYVQKEGIAVTAYSSFGPLS
FLELGHQVAKDTPLLFEHSTVKSIAEKHGKTPAQVLLRWATQRNIAVIPKSNNPGRLAQNLDVTAWDLEPADI
EALSALNKNLRFNNPPSYGLYIPIFA

### SEQ ID Nr. 4:

ATGGCCACGCCTACTATCAAGCTGAACAGCGGCTATGACATGCCCCTGGTGGGCTTTGGTCTGTGGAAG
GTCAACAAGGAAACCTGCGCGGACCAGGTCTACGAGGCTATCAAGGCGGGCTACCGCTTGTTTGACGGT
GCGTGCGACTATGGCAACGAAGTTGAGGCCGGCCAGGGTGTCGCTCGCGCCATCAAGGAAGGCATTGT
GAAGCGTGAGGACCTCTTCATTGTGTCCAAGCTGTGGAACACGTTCCACGAGGCCGACAAGGTCGAGCC
GATCGCGCGGAAGCAGCTGGCCGACTGGGGCCTCGACTACTTTGACCTGTACCTCATCCACTTCCCGATC
GCGCTGAAGTACGTCGACCCGGCCGAGATCTACCCGCCGGGCTGGACGGGCACCAAGAAGGAGGTCGA
GTTCAGCAACGCGACGATCCAGGAGACGTGGCAGGCCATGGAGACCCTGGTCGACAAGAAGCTGACGC
GCAGCATCGGCATCAGCAACTTCAGCGCCCAGCTGATCATGGACCTGCTGCGGTACGCGCGCATCCGCCC
CGCGACCTTGCAGATCGAGCACCACCCGTACCTGACGCAGCAGGCGCTGGTCGAGTACGTGCAGAAGGA
GGGCATCGCCGTGACGGCGTACTCGTCCTTCGGCCCACTGAGCTTCCTGGAACTGGGCCACCAGGTCGCC
AAGGACACGCCGCTGCTCTTCGAGCACTCGACCGTCAAGTCGATCGCCGAGAAGCACGGCAAGACGCCC
GCCCAGGTGCTACTGCGCTGGGCCACCCAGCGCAACATCGCCGTCATCCCCAAGAGCAACAACCCGGGC
CGCCTGGCGCAGAACCTGGACGTGACGGCGTGGGATCTGGAGCCCGCCGACATTGAGGCCTTGAGCGC
GCTGAACAAGAACCTTCGATTCAACAACCCACCTAGCTACGGACTGTACATCCCGATCTTCGCTTAA

### SEQ ID Nr. 5:

MAPVIKLNSGYDMPQVGFGLWKVDNAVASDVVYNAIKAGYRLFDGACDYGNEVECGQGVARAISEGIVKRE
DLFIVSKLWNTFHDAERVEPIVKKQLADWGIEYFDLYLIHFPVALEWVDPAVRYPPGWHYDGKEEIRPSKATIQ
ETWTALESLVSKGLSKSIGISNFQAQLIYDLLRYAKIRPATLQVEHHPYLVQQELINLAKREGIAVTAYSSFGPASF
KEFNMKHADALAPLIEDETIKKIAAKHNRPASQVLLRWATQRGLAIIPKSTRPQIMAENFQSIDFDLSEEDIATIS
AFDRGIRFNQPSNYFPTELLWIFG

### SEQ ID Nr. 6:

ATGGCGCCGGTGATTAAACTGAACAGCGGCTATGATATGCCGCAGGTGGGCTTTGGCCTGTGGAAAGTG
GATAACGCGGTGGCGAGCGATGTGGTGTATAACGCGATTAAAGCGGGCTATCGCCTGTTTGATGGCGCG
TGCGATTATGGCAACGAAGTGGAATGCGGCCAGGGCGTGGCGCGCGCGATTAGCGAAGGCATTGTGAA
ACGCGAAGATCTGTTTATTGTGAGCAAACTGTGGAACACCTTTCATGATGCGGAACGCGTGGAACCGATT
GTGAAAAAACAGCTGGCGGATTGGGGCATTGAATATTTTGATCTGTATCTGATTCATTTTCCGGTGGCGC
TGGAATGGGTGGATCCGGCGGTGCGCTATCCGCCGGGCTGGCATTATGATGGCAAAGAAGAAATTCGCC
CGAGCAAAGCGACCATTCAGGAAACCTGGACCGCGCTGGAAAGCCTGGTGAGCAAAGGCCTGAGCAAA
AGCATTGGCATTAGCAACTTTCAGGCGCAGCTGATTTATGATCTGCTGCGCTATGCGAAAATTCGCCCGG
CGACCCTGCAGGTGGAACATCATCCGTATCTGGTGCAGCAGGAACTGATTAACCTGGCGAAACGCGAAG
GCATTGCGGTGACCGCGTATAGCAGCTTTGGCCCGGCGAGCTTTAAAGAATTTAACATGAAACATGCGG
ATGCGCTGGCGCCGCTGATTGAAGATGAAACCATTAAAAAAATTGCGGCGAAACATAACCGCCCGGCGA
GCCAGGTGCTGCTGCGCTGGGCGACCCAGCGCGGCCTGGCGATTATTCCGAAAAGCACCCGCCCGCAGA
TTATGGCGGAAAACTTTCAGAGCATTGATTTTGATCTGAGCGAAGAAGATATTGCGACCATTAGCGCGTT
TGATCGCGGCATTCGCTTTAACCAGCCGAGCAACTATTTTCCGACCGAACTGCTGTGGATTTTTGGCTAA

### SEQ ID Nr. 7:

MSTTVNTPTIKLNSGYEMPLVGFGCWKVTNATAADQIYNAIKTGYRLFDGAEDYGNEKEVGEGINRAIKDGL
VKREELFITSKLWNNFHDPKNVETALNKTLSDLNLDYVDLFLIHFPIAFKFVPIEEKYPPGFYCGDGDNFHYEDV
PLLDTWKALEKLVEAGKIKSIGISNFTGALIYDLIRGATIKPAVLQIEHHPYLQQPKLIEYVQKAGIAITGYSSFGPQ
SFLELESKRALNTPTLFEHETIKSIADKHGKSPAQVLLRWATQRNIAVIPKSNNPERLAQNLSVVDFDLTKDDLD
NIAKLDIGLRFNDPWDWDNIPIFV

### SEQ ID Nr. 8:

ATGAGCACGACCGTTAATACTCCGACGATCAAACTGAACTCCGGTTACGAGATGCCGCTGGTTGGCTTTG
GCTGTTGGAAAGTTACCAATGCGACGGCGGCGGACCAGATTTATAACGCTATTAAAACCGGTTACCGTCT
GTTCGATGGCGCAGAGGACTACGGTAACGAGAAAGAAGTGGGTGAAGGTATTAATCGCGCAATTAAAG
ACGGTCTGGTCAAACGTGAAGAGCTGTTTATTACCTCGAAACTGTGGAATAACTTCCATGATCCTAAGAA
CGTGGAGACTGCGCTGAATAAGACCCTGAGCGATCTGAACCTGGACTATGTGGATCTGTTTCTGATTCAC
TTCCCGATTGCTTTCAAATTTGTCCCGATCGAAGAAAAGTACCCACCGGGCTTCTACTGCGGTGACGGCG
ACAACTTCCATTATGAGGATGTTCCGCTGCTGGACACGTGGAAGGCGCTGGAGAAATTGGTGGAAGCCG
GTAAGATCAAGTCCATTGGCATTAGCAACTTCACCGGTGCCTTGATTTACGATTTGATCCGTGGTGCGACC
ATTAAACCGGCGGTTCTGCAGATCGAGCATCACCCGTATCTGCAACAGCCGAAACTGATCGAATACGTTC
AGAAAGCAGGTATCGCCATCACTGGCTATAGCAGCTTCGGTCCACAGAGCTTCCTGGAGCTGGAGAGCA
AGCGTGCCCTGAATACCCCGACGTTGTTTGAACACGAAACCATCAAGTCTATCGCTGACAAACACGGTAA
GAGCCCTGCACAAGTCCTGCTGCGCTGGGCAACGCAACGTAATATTGCGGTTATTCCGAAGAGCAATAAC

CCGGAGCGTCTGGCGCAGAATCTGTCTGTCGTCGACTTTGATTTGACCAAGGATGACCTGGATAATATCG
CGAAGCTGGACATCGGCCTGCGCTTCAACGATCCGTGGGATTGGGACAACATCCCGATCTTTGTGTA

### SEQ ID Nr. 9:

MTYLAETVTLNNGEKMPLVGLGCWKMPNDVCADQIYEAIKIGYRLFDGAQDYANEKEVGQGVNRAIKEGLV
KREDLVVVSKLWNSFHHPDNVPRALERTLSDLQLDYVDIFYIHFPLAFKPVPFDEKYPPGFYTGKEDEAKGHIEE
EQVPLLDTWRALEKLVDQGKIKSLGISNFSGALIQDLLRGARIKPVALQIEHHPYLTQERLIKYVKNAGIQVVAYS
SFGPVSFLELENKKALNTPTLFEHDTIKSIASKHKVTPQQVLLRWATQNGIAIIPKSSKKERLLDNLRINDALTLTD
DELKQISGLNQNIRFNDPWEWLDNEFPTFI

### SEQ ID Nr. 10:

ATGACGTACTTAGCAGAAACAGTTACTTTAAACAATGGCGAAAAGATGCCGCTAGTCGGCTTAGGTTGCT
GGAAGATGCCCAACGACGTTTGTGCCGACCAAATTTACGAAGCCATTAAGATCGGATATCGTTTATTCGA
TGGTGCCCAAGATTACGCCAACGAGAAAGAAGTTGGACAGGGTGTCAACAGAGCCATCAAAGAAGGGC
TTGTTAAGAGAGAGGATTTAGTTGTTGTCTCCAAGCTATGGAACAGTTTCCACCATCCGGACAACGTACC
TCGTGCTTTGGAAAGAACTCTTTCCGATTTGCAATTGGACTATGTTGACATATTCTACATCCATTTCCCATT
GGCCTTCAAGCCTGTGCCATTCGATGAGAAGTATCCTCCAGGTTTCTACACCGGTAAGGAAGACGAAGCT
AAAGGTCACATTGAAGAAGAACAAGTACCACTATTGGACACTTGGAGGGCTTTAGAAAAACTAGTCGAC
CAAGGTAAGATCAAGTCCTTGGGTATTTCCAACTTTTCAGGTGCGTTGATCCAAGATTTGCTACGTGGTGC
TCGTATCAAGCCAGTTGCCCTACAAATCGAGCATCATCCATATTTGACACAGGAAAGATTGATTAAATAC
GTCAAGAATGCCGGCATTCAAGTGGTTGCCTACTCCTCGTTCGGTCCAGTGTCGTTCCTGGAATTGGAAA
ACAAGAAAGCTCTTAACACCCCTACTTTGTTTGAGCATGACACGATTAAGTCTATCGCTTCCAAGCACAAA
GTCACTCCACAACAAGTTCTGTTGAGATGGGCCACACAAAATGGCATTGCCATCATTCCAAAATCATCAA
AGAAGGAAAGATTGCTGGATAACTTGAGGATCAACGATGCATTGACTTTGACCGATGATGAATTGAAGC
AAATTTCCGGCTTGAATCAAAATATCAGATTCAATGACCCTTGGGAATGGCTAGACAACGAATTCCCAAC
TTTCATCTAA

### SEQ ID Nr. 11:

MPSPVIKLNSGYDMPQVGFGLWKVDNSVAADVVYNAIKAGYRLFDGACDYGNEVECGKGIARAISEGIVKRE
DLFIVSKLWNTFHDAERVEPIVKKQLADWGIDYFDLYLIHFPVALEYVDPSVRYPPGWFYDGKSEIRTSKATIQE
TWTAMESLVAKGLTKSIGISNFQAQLIYDLLRYAKIRPATLQIEHHPYLVQQELLNLAKQEGIAVTAYSSFGPASF
LEFNMQHAVKLTPLIEDETIKKIAAKYNREPAQVLLRWATQRGLAVIPKTTRPALMASNFQCTDFDLSEEDIATI
SSFDRGIRFNQPSNYFPTEYLWIFG

### SEQ ID Nr. 12:

ATGCCGTCACCTGTCATCAAGCTCAACAGCGGCTATGACATGCCCCAGGTGGGCTTCGGCCTCTGGAAGG
TCGACAATTCCGTTGCCGCGGACGTCGTCTACAACGCCATCAAGGCCGGCTACCGCCTCTTCGATGGCGC
TTGCGACTACGGCAACGAGGTCGAGTGCGGCAAGGGTATCGCGCGCGCCATCTCGGAGGGCATTGTGA
AGCGTGAGGACCTCTTCATCGTGTCCAAGCTCTGGAACACCTTCCACGACGCCGAGCGCGTCGAGCCCAT
CGTCAAGAAGCAGCTCGCCGACTGGGGCATTGACTACTTCGACCTCTACCTCATCCACTTCCCTGTCGCCC
TCGAGTACGTCGACCCCTCGGTCCGCTACCCGCCCGGTTGGTTCTACGATGGCAAGAGCGAGATCCGCAC
CAGCAAGGCCACCATCCAGGAGACCTGGACTGCCATGGAGTCCCTTGTCGCCAAGGGCCTCACCAAGAG
CATCGGTATTTCCAACTTCCAGGCCCAGCTGATCTACGACCTCCTGCGGTACGCCAAGATCCGCCCGGCC
ACCCTCCAGATCGAGCACCACCCCTACCTCGTCCAGCAGGAGCTGCTCAACCTCGCCAAGCAGGAGGGCA
TCGCCGTCACCGCCTACAGCTCCTTCGGCCCCGCCAGCTTCCTCGAGTTCAACATGCAGCACGCCGTCAAG
CTCACCCCCCTTATCGAGGACGAGACCATCAAGAAGATCGCCGCCAAGTACAACCGCGAGCCCGCCCAA
GTCCTGCTCCGCTGGGCCACTCAGCGTGGCCTGGCCGTCATCCCCAAGACCACCCGCCCCGCCCTCATGG
CCTCCAACTTCCAGTGCACCGACTTTGATCTCTCCGAGGAGGACATCGCCACCATCTCGTCCTTCGACCGC
GGCATTCGCTTCAACCAGCCCTCCAACTACTTCCCCACCGAGTATCTCTGGATCTTTGGCTAA

### SEQ ID Nr. 13:

MSTDTILIPGIDTPVSRVALGTWAIGGWMWGGPDDGNGVRTIHAALDDGINLIDTAPVYGFGHSEEVVGRA
LAEKPHKAFVATKLGLNWTDDKPEDRKVFRDSRPARIRQEVEDSLRRLRVETIDLEQIHWPDAKTPIDESAREL
QKLHQEGKIRALGVSNFSPEQMDIFREVAPLATIQPPLNLFERTCEKDILPYAKKNDAVVLAYGSLCRGLLSGK
MSKDTTFPKDDLRSGDPKFQKPKFESYLAAVEDFKKLAAKRNKSVLAFAVRWVLDQGPVIALWGARKPEQVS
GVRDVFGWSLTDEEKKAVDEILAHHVPEAIDPTFMGPPNRD

### SEQ ID Nr. 14:

ATGTCCACCGACACCATTCTCATTCCAGGCATTGATACGCCCGTTTCTCGCGTTGCCCTTGGCACCTGGGC
TATCGGTGGCTGGATGTGGGGCGGCCCTGACGATGGAAACGGCGTCCGCACCATTCATGCCGCCCTTGA
TGATGGCATCAATCTGATTGATACCGCTCCGGTTTACGGCTTTGGACATTCCGAAGAAGTTGTGGGTCGC
GCACTGGCTGAGAAGCCGCACAAGGCCTTTGTCGCCACAAAGCTGGGCCTGAACTGGACGGATGACAAG
CCGGAAGACCGGAAGGTCTTTCGTGACTCCCGCCCCGCCCGTATCCGACAGGAAGTGGAAGACTCCCTG
CGCCGCCTGCGCGTTGAGACGATCGACCTTGAGCAGATTCACTGGCCGGATGCCAAGACCCCGATCGAC
GAAAGCGCGCGCGAGCTTCAGAAGCTGCATCAGGAAGGCAAGATCCGCGCTCTGGGCGTCAGCAACTTC
TCGCCCGAACAGATGGATATTTTCCGCGAAGTCGCGCCCCTCGCCACGATCCAGCCGCCGCTGAACCTGT
TCGAGCGTACCTGTGAAAAGGACATCCTGCCGTACGCGAAGAAGAACGATGCGGTTGTTCTGGCCTATG
GTTCGCTATGTCGCGGTCTGCTGTCCGGCAAAATGAGCAAGGACACGACGTTCCCGAAGGATGATCTGC
GTTCGGGTGATCCGAAATTCCAGAAGCCGAAGTTCGAGAGCTATCTGGCCGCTGTCGAGGACTTCAAGA
AGCTGGCTGCCAAGCGGAACAAGTCCGTTCTCGCCTTTGCTGTCCGCTGGGTACTGGATCAGGGACCGG

TCATTGCTCTCTGGGGCGCTCGCAAGCCGGAGCAGGTCTCAGGCGTACGAGACGTTTTCGGCTGGTCTCT
GACGGATGAAGAAAAGAAGGCCGTAGACGAAATTCTGGCGCATCATGTGCCGGAGGCCATCGACCCGA
CCTTTATGGGGCCGCCGAACCGCGACTAA

### SEQ ID Nr. 15:

MSSDTILIPGIDTPVSRVALGTWAIGGWMWGGPDDENGVRTIHAALDDGINFIDTAPVYGFGHSEEVVGRA
LAEKPNKAFVATKLGLNWTDDKPEDRKVFRDSRPARIRKEVEDSLRRLRVETIDLEQIHWPDDKTPIDESAREL
QKLHQEGKIRALGVSNFSPEQMDIFREVAPLATIQPPLNLFERTCEKDILPYAKKNNAVVLAYGALCRGLLSGK
MNKDTSFPKDDLRSGDPKFQKPKFESYLAAVEDFKELAAKRDKSVLAFAVRWVLDQGPVIALWGARKPEQVS
GVKDVFGWSLTDEEKKAVDEILARHVPDAIDPTFMGPPNRD

### SEQ ID Nr. 16:

ATGTCCTCAGACACCATTCTCATTCCCGGCATCGACACTCCGGTTTCCCGCGTGGCTCTCGGCACCTGGGC
CATTGGCGGCTGGATGTGGGGCGGGCCGGACGATGAAAACGGCGTTCGCACCATCCATGCAGCTCTCGA
TGACGGCATCAATTTCATCGACACGGCACCCGTCTATGGCTTCGGGCACTCAGAAGAAGTTGTTGGCCGG
GCACTGGCCGAAAAGCCCAACAAGGCTTTCGTTGCGACAAAGCTGGGCCTGAACTGGACGGATGACAAA
CCCGAAGACCGCAAGGTTTTCCGTGATTCCCGCCCTGCCCGCATCCGCAAGGAAGTGGAAGACTCCCTGC
GCCGCCTGCGGGTCGAAACGATTGATCTCGAACAGATCCATTGGCCCGATGACAAGACTCCCATCGATGA
AAGCGCACGCGAGCTGCAGAAACTTCATCAGGAAGGCAAGATCCGGGCGCTCGGCGTTAGCAATTTCTC
GCCGGAGCAGATGGATATCTTTCGCGAAGTCGCCCCACTCGCAACAATTCAGCCCCCGCTGAACCTGTTC
GAACGCACCTGCGAAAAAGATATTCTCCCATACGCCAAGAAGAATAACGCCGTGGTGCTCGCTTATGGC
GCGCTCTGCCGCGGACTGCTCTCCGGCAAGATGAACAAGGATACGAGCTTCCCGAAAGATGACCTGCGT
TCAGGTGATCCGAAGTTCCAGAAGCCAAAGTTCGAGAGCTATCTCGCCGCAGTCGAAGACTTCAAGGAG
CTGGCCGCCAAGCGCGACAAGTCCGTTCTCGCCTTCGCTGTACGCTGGGTTCTGGATCAGGGGCCAGTCA
TCGCACTCTGGGGCGCACGCAAGCCTGAACAGGTCTCCGGCGTGAAGGATGTTTTCGGCTGGTCTCTGA
CCGACGAAGAAAAGAAGGCTGTGGACGAAATTCTGGCACGTCACGTCCCGGACGCGATCGACCCGACCT
TCATGGGGCCACCGAACCGCGACTAA

### SEQ ID Nr. 17:

MSSLVTLNNGLKMPLVGLGCWKIDKKVCANQIYEAIKLGYRLFDGACDYGNEKEVGEGIRKAISEGLVSRKDIF
VVSKLWNNFHHPDHVKLALKKTLSDMGLDYLDLYYIHFPIAFKYVPFEEKYPPGFYTGADDEKKGHITEAHVPII
DTYRALEECVDEGLIKSIGVSNFQGSLIQDLLRGCRIKPVALQIEHHPYLTQEHLVEFCKLHDIQVVAYSSFGPQS
FIEMDLQLAKTTPTLFENDVIKKVSQNHPGSTTSQVLLRWATQRGIAVIPKSSKKERLLGNLEIEKKFTLTEQELK
DISALNANIRFNDPWTWLDGKFPTFA

### SEQ ID Nr. 18:

ATGTCTTCACTGGTTACTCTTAATAACGGTCTGAAAATGCCCCTAGTCGGCTTAGGGTGCTGGAAAATTG
ACAAAAAAGTCTGTGCGAATCAAATTTATGAAGCTATCAAATTAGGCTACCGTTTATTCGATGGTGCTTGC
GACTACGGCAACGAAAAGGAAGTTGGTGAAGGTATCAGGAAAGCCATCTCCGAAGGTCTTGTTTCTAGA
AAGGATATATTTGTTGTTTCAAAGTTATGGAACAATTTTCACCATCCTGATCATGTAAAATTAGCTTTAAA
GAAGACCTTAAGCGATATGGGACTTGATTATTTAGACCTGTATTATATTCACTTCCCAATCGCCTTCAAAT
ATGTTCCATTTGAAGAGAAATACCCTCCAGGATTCTATACGGGCGCAGATGACGAGAAGAAAGGTCACA
TCACCGAAGCACATGTACCAATCATAGATACGTACCGGGCTCTGGAAGAATGTGTTGATGAAGGCTTGAT
TAAGTCTATTGGTGTTTCCAACTTTCAGGGAAGCTTGATTCAAGATTTATTACGTGGTTGTAGAATCAAGC
CCGTGGCTTTGCAAATTGAACACCATCCTTATTTGACTCAAGAACACCTAGTTGAGTTTTGTAAATTACAC
GATATCCAAGTAGTTGCTTACTCCTCCTTCGGTCCTCAATCATTCATTGAGATGGACTTACAGTTGGCAAA
AACCACGCCAACTCTGTTCGAGAATGATGTAATCAAGAAGGTCTCACAAAACCATCCAGGCAGTACCACT
TCCCAAGTATTGCTTAGATGGGCAACTCAGAGAGGCATTGCCGTCATTCCAAAATCTTCCAAGAAGGAAA
GGTTACTTGGCAACCTAGAAATCGAAAAAAAGTTCACTTTAACGGAGCAAGAATTGAAGGATATTTCTGC
ACTAAATGCCAACATCAGATTTAATGATCCATGGACCTGGTTGGATGGTAAATTCCCCACTTTTGCCTAA

Die Oxidoreduktase zur Oxidation von Allitol zu Allose umfasst oder besteht vorzugsweise aus einer Aminosäuresequenz, die eine Identität zu SEQ ID Nr. 1, SEQ ID Nr. 3, SEQ ID Nr. 5, SEQ ID Nr. 7, SEQ ID Nr. 9, SEQ ID Nr. 11, SEQ ID Nr. 13, SEQ ID Nr. 15 oder SEQ ID Nr. 17 von mindestens 75%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die erfindungsgemäße Oxidoreduktase zur Oxidation von Allitol zu Allose die Aminosäuresequenz SEQ ID Nr. 1, SEQ ID Nr. 3, SEQ ID Nr. 5, SEQ ID Nr. 7, SEQ ID Nr. 9, SEQ ID Nr. 11, SEQ ID Nr. 13, SEQ ID Nr. 15 oder SEQ ID Nr. 17 oder besteht aus dieser.

Alternativ, umfasst oder besteht die Oxidoreduktase zur Oxidation von Allitol zu Allose vorzugsweise aus einer Aminosäuresequenz, die durch eine Nukleinsäure kodiert wird, die eine Identität zu SEQ ID Nr. 2, SEQ ID Nr. 4, SEQ ID Nr. 6, SEQ ID Nr. 8, SEQ ID Nr. 10, SEQ ID Nr. 12, SEQ ID Nr. 14, SEQ ID Nr. 16 oder SEQ ID Nr. 18 von mindestens 75%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die Nukleinsäure, welche die erfindungsgemäße Oxidoreduktase zur Oxidation von Allitol zu Allose kodiert, die Nukleinsäuresequenz SEQ ID Nr. 2, SEQ ID Nr. 4, SEQ ID Nr. 6, SEQ ID Nr. 8, SEQ ID Nr. 10, SEQ ID Nr. 12, SEQ ID Nr. 14, SEQ ID Nr. 16 oder SEQ ID Nr. 18 oder besteht aus dieser.

Der Begriff "Identität", wie hier verwendet, bezieht sich auf den Prozentsatz der identischen Nukleotid- bzw. Aminosäureübereinstimmungen zwischen mindestens zwei, unter Verwendung eines standardisierten Algorithmus miteinander ausgerichteten Nukleotid- bzw. Aminosäuresequenzen ("Alignment"). Ein solcher Algorithmus kann, in einer standardisierten und reproduzierbaren Weise, Lücken ("Gap") in die verglichenen Sequenzen einfügen, um das Alignment zwischen zwei Sequenzen zu optimieren, und somit einen aussagekräftigeren Vergleich der beiden Sequenzen erreichen.

Die prozentuale Identität zwischen Sequenzen kann unter Verwendung von ein oder mehreren Computeralgorithmen oder im Stand der Technik bekannten oder hierin beschriebenen Programmen bestimmt werden. Erfindungsgemäß wird zur Bestimmung der Identität das durch National Center for Biotechnology Information (NCBI) bereitgestellte Basic Local Alignment Search Tool (BLAST) (Altschul et al., 1990), verwendet. Die BLAST-Software-Reihe enthält verschiedene Programme, einschließlich ein als "BLAST 2 Sequences" genanntes Werkzeug, das für den direkten paarweisen Vergleich von zwei Nukleotid- bzw. Aminosäuresequenzen verwendet wird. "BLAST 2 Sequences" kann auch über die NCBI World Wide Web-Seite im Internet interaktiv abgerufen und verwendet werden. Das blastn-Programm (für Nukleotidsequenzen) verwendet als Vorgaben eine Wortlänge (W) von 28, eine Erwartung (E) von 0,05, M = 1, N = -2 und einen Vergleich beider Stränge. Für Aminosäuresequenzen verwendet das blastp-Programm als Vorgaben eine Wortlänge von 3 und eine Erwartung (E) von 0,05 und die BLOSUM62-Scoring-Matrix (Henikoff & Henikoff, 1989), Alignments (B) von 50, Erwartung (E) von 0,05, M = 1, N =-2.

Alternativ, umfasst die Oxidoreduktase zur Oxidation von Allitol zu Allose vorzugsweise eine Aminosäuresequenz oder besteht aus dieser, die durch eine Nukleinsäure kodiert wird, die unter stringenten Bedingungen an ein Nukleinsäuremolekül mit der Nukleinsäuresequenz SEQ ID Nr. 2, SEQ ID Nr. 4, SEQ ID Nr. 6, SEQ ID Nr. 8, SEQ ID Nr. 10, SEQ ID Nr. 12, SEQ ID Nr. 14, SEQ ID Nr. 16 oder SEQ ID Nr. 18 bindet. Wie hierin verwendet, beziehen sich die stringenten Bedingungen auf Bedingungen, unter denen sogenannte spezifische Hybride, jedoch keine unspezifischen Hybride gebildet werden.

Beispielsweise kann die Hybridisierung durch herkömmlich bekannte Verfahren durchgeführt werden, wie die von J. Sambrook et al. in Molecular Cloning, A Laboratory Manual, 2nd Ed., Cold Spring Harbor Laboratory (1989) beschriebenen. Unter stringenter Bedingung ist eine Bedingung gemeint, bei der das Waschen bei 65 °C und einer Salzkonzentration von 0,1x bis 2x SSC durchgeführt wird (wobei unter 1xSSC eine Mischung aus 0,15 M Natriumchlorid/0,015 M Natriumcitrat zu verstehen ist).

Die zur Kofaktor-Regeneration eingesetzte Alkoholdehydrogenase (ADH) kann aus einer der Gruppen EC 1.1.1.1 (NAD-abhängige ADH) und EC 1.1.1.2 (NADP-abhängige ADH) stammen.

Die zur Kofaktor-Regeneration eingesetzte NAD(P)H-Oxidase kann aus einer der Gruppen EC 1.6.3.1 (NAD(P)H-Oxidase (H₂O₂-bildend)), EC 1.6.3.2 (NAD(P)H-Oxidase (H₂O-bildend)), EC 1.6.3.3 (NADH-Oxidase (H₂O₂-bildend)) sowie EC 1.6.3.4 (NADH-Oxidase (H₂O-bildend)) stammen, wobei die H₂O-bildenden Klassen besonders bevorzugt sind.

Die hier vorgestellte enzymatische Strategie in Kombination mit der Kofaktor-Regeneration ermöglicht einen biokatalytischen, umweltfreundlichen und hocheffizienten Produktionsprozess zur Herstellung von Allose.

### Materialien

D-Psicose wurde von TCI und Hunan Garden Naturals Inc. (China), D-Allose und Allitol wurden von TCI, D-Fructose, Lysozym, NADPH-Tetranatriumsalz, und Methanol wurden von PanReac AppliChem (ITW Reagents), Zinkchlorid, IPTG (Isopropyl-β-D-thiogalactopyranosid) wurden von Sigma-Aldrich, Magnesiumchlorid-Hexahydrat, Kaliumdihydrogenphosphat, di-Kaliumhydrogenphosphat, NAD⁺, NADH-Dinatriumsalz, NADP⁺-Dinatriumsalz sowie Natriumdodecylsulfat (SDS) wurden von Carl Roth und Triethanolamin (TEA) wurde von Chem-Lab NV bezogen.

### Produktion der Enzyme & Herstellung der Lysate

### Allgemeines zur Expression von rekombinanten Enzymen in E. coli

Für die rekombinante Enzymproduktion in einem *Escherichia coli*-Stamm wurde zunächst das zu exprimierende Gen in einer PCR unter der Verwendung der genomischen DNA oder deren synthetisch an die Codon-Verwendung von *E. coli* angepasstes Äquivalent als Matrize zusammen mit spezifischen Oligonukleotiden, die zusätzlich Erkennungssequenzen für Restriktionsendonukleasen tragen, amplifiziert und aus dem Reaktionsgemisch isoliert. Nach dem Nukleinsäure-Verdau mit den Restriktionsenzymen Sphl und Hindlll wurde das für das Target-Enzym kodierende Genfragment in das mit Sphl und Hindlll geschnittene Rückgrat des Expressionsvektors pQE70-Kan ligiert. Das Ligationsprodukt wurde in chemisch kompetente *E*. *coli*-Zellen Top10F transformiert und die entstandenen Kolonien wurden für die Plasmid-Isolierung und Restriktionsanalyse benutzt.

Das Ergebnis des Klonierungs-Schritts wurde mittels Restriktionsenzym-Verdau und DNA-Sequenzierung überprüft. Das resultierende Konstrukt trägt das Target-Gen unter dem IPTGinduzierbaren T5-Promotor.

Für die Überexpression des Enzymes in *E. coli* wurde das resultierende Expressionsplasmid in die kompetenten Expressionszellen RB791 transformiert. Nach 24 h Inkubation bei 37 °C wurden entstandene Kolonien für die Expressionstests in LB-Medium angeimpft.

Am nächsten Tag wurden damit Expressionskulturen mit einer optische Dichte OD₅₅₀ von 0,02 angeimpft und bei 37 °C geschüttelt, bis eine OD₅₅₀ von 0,3 erreicht wurde. Anschließend wurde die Temperatur auf 25 °C gesenkt und die Kulturen wurden beim Erreichen einer OD₅₅₀ von 0,5 mit 0,1 mM IPTG induziert. Nach 22 h wurden die Kulturen geerntet (vom Medium mittels Zentrifugation in Form eines Zellpellets abgetrennt) und auf die Expression des rekombinanten Enzymes mit Hilfe von SDS-Gelelektrophorese und einer Aktivitätsbestimmung (Verwendung in Use-Test oder optisch-enzymatischer Assay) analysiert.

### Herstellung von Zell-Lysaten mittels GEA-Aufschlusses

Zur Herstellung einer Zell-Suspension (Volumen > 50 ml) wurde das nach obigen Verfahren hergestellte Zellpellet in einem geeigneten Gefäß eingewogen und in Puffer (Triethanolamin (TEA) - HCl oder Kaliumphosphat-Puffer (KPP); siehe Tabelle 1 für verwendete Puffer-Systeme) gelöst. Der Massenanteil an Biomasse beträgt üblicherweise 20%, der Rest entfällt auf den Puffer.

Zum Zell-Aufschluss wurde ein Lab Homogenizer PandaPLUS 2000 von GEA verwendet (Druck ca. 1000 bar).

Das erhaltene Homogenat wurde 10 min lang bei 4 °C und 16000 rpm zentrifugiert (Hermle Z 446 K Zentrifuge), um die unlöslichen Zellfragmente abzutrennen und das Lysat zu erhalten.

### Herstellung von Zell-Lysaten mittels Retsch-Aufschlusses

Zur Herstellung einer Zell-Suspension wurde das nach obigen Verfahren hergestellte Zellpellet (ca. 50 mg) in einem 2 ml Eppendorf-Vial eingewogen und mit Puffer (ca. 450 µl) versetzt (Tabelle 1 für verwendete Puffer-Systeme) und mittels Vortexens gelöst. Dadurch wird eine 10%ige Zell-Suspension erhalten.

Die Suspension wurde mit Glasperlen versetzt und in einer Retsch-Mühle MM 400 (24 Hz, 10 min) aufgeschlossen. Im Anschluss wurde das Homogenat für 1 min bei 4 °C und 16000 rpm zentrifugiert (Himac Zentrifuge CT 15RE), um das Lysat zu erhalten.

### Herstellung von Zell-Lysaten mittels Sonifier-Aufschlusses

Zur Herstellung einer Zell-Suspension wurde das nach obigen Verfahren hergestellte Zellpellet in einem geeigneten Gefäß eingewogen und mit Puffer und Lysozym (finale Konzentration 0,5 mg/ml) versetzt (siehe Tabelle 1 für verwendete Puffer-Systeme) und unter Rührung gelöst. Der Massenanteil an Biomasse beträgt üblicherweise 20%, der Rest entfällt auf den Puffer.

Zum Zell-Aufschluss wurde ein Branson Sonifier 450 verwendet. Die Suspension wurde dreimal mit je 15 Ultraschall-Stößen (Einstellungen am Gerät: Timer = 15; Duty Cycle = 50; Output Control = 3 - 5) behandelt.

Das erhaltene Homogenat wurde 10 min lang bei 4 °C und 16000 rpm zentrifugiert (Eppendorf Zentrifuge 5417R), um die unlöslichen Zellfragmente abzutrennen und das Lysat zu erhalten.

**Tabelle 1. Enzymklassen, Spenderorganismen und Aufschlussbedingungen für die in den Beispielen verwendeten Enzyme.**

| **Enzymtyp (EC-Klasse)** | **katalysierte Reaktion** | **Spenderorganismus** | **Aufschlussbedingungen** | **Literatur** |
|---|---|---|---|---|
| D-Psicose-3-Epimerase (EC 5.1.3.30) | D-Fructose → D-Psicose | *Clostridium cellulolyticum* H10 | GEA (20% Biomasse in 100 mM TEA-HCl pH 7 + 2 mM MgCl₂) | (Mu et al., 2011; Chan et al., 2012) |
| Ribitol-Dehydrogenase (EC 1.1.1.56) | D-Psicose → Allitol | *Klebsiella pneumoniae* | GEA (20% Biomasse in 100 mM TEA-HCl pH 7) | (Takeshita et al., 2000; Sequenz in NCBI Protein Database: WP_265716617.1) |
| Xylose-Reduktase I | Allitol → Allose | *Thermochaetoides thermophila* DSM 1495 | Retsch (10% Biomasse in 100 mM TEA-HCl pH 7) | Aminosäuren: SEQ ID Nr. 1; Nucleotide: SEQ ID Nr. 2 |
| Xylose-Reduktase II | Allitol → Allose | *Rasamsonia emersonii* | Retsch (10% Biomasse in 100 mM TEA-HCl pH 7) | Aminosäuren: SEQ ID Nr. 3; Nucleotide: SEQ ID Nr. 4 |
| Xylose-Reduktase III | Allitol → Allose | *Chaetomium globosum* CBS 148.51 | Retsch (10% Biomasse in 100 mM TEA-HCl pH 7) | Aminosäuren: SEQ ID Nr. 5; Nucleotide: SEQ ID Nr. 6 |
| Xylose-Reduktase IV | Allitol → Allose | *Candida tropicalis* | Retsch (10% Biomasse in 100 mM TEA-HCl pH 7) | Aminosäuren: SEQ ID Nr. 7; Nucleotide: SEQ ID Nr. 8 |
| Xylose-Reduktase V | Allitol → Allose | *Saccharomyces cerevisiae* | Retsch (10% Biomasse in 100 mM TEA-HCl pH 7) | Aminosäuren: SEQ ID Nr. 17; Nucleotide: SEQ ID Nr. 18 |
| Aldo/Keto-Reduktase I | Allitol → Allose | *Kluyveromyces lactis* | Sonifier (20% Biomasse in 100 mM TEA-HCl pH 7); Retsch (10% Biomasse in 100 mM TEA-HCl pH 7) | Aminosäuren: SEQ ID Nr. 9; Nucleotide: SEQ ID Nr. 10 |
| Aldo/Keto-Reduktase II | Allitol → Allose | *Thermothelomyces thermophilus* ATCC 42464 | Retsch (10% Biomasse in 100 mM TEA-HCl pH 7) | Aminosäuren: SEQ ID Nr. 11; Nucleotide: SEQ ID Nr. 12 |
| Aldo/Keto-Reduktase III | Allitol → Allose | *Gluconobacter frateurii* | Retsch (10% Biomasse in 100 mM TEA-HCl pH 7) | Aminosäuren: SEQ ID Nr. 13; Nucleotide: SEQ ID Nr. 14 |
| Aldo/Keto-Reduktase IV | Allitol → Allose | *Gluconobacter cerinus* NRIC 0229 | Retsch (10% Biomasse in 100 mM TEA-HCl pH 7) | Aminosäuren: SEQ ID Nr. 15; Nucleotide: SEQ ID Nr. 16 |
| Alkoholdehydro genase (ADH) (EC 1.1.1.1) | 2-Propanol → Aceton | *(Geo-)Bacillus stearothermophilus* NCA1503 | GEA (20% Biomasse in 100 mM TEA-HCl pH 7 + 0,5 mM ZnCl₂) | (Sakoda & Imanaka, 1992) |
| NAD(P)H-Oxidase (EC 1.6.3.2) | NAD(P)H → NAD(P)⁺ | *Streptococcus mutans*; Mutante | GEA (20% Biomasse in 100 mM KPP pH 7) | (Petschacher et al., 2014) |

### Analytische Methoden

### High Performance Liquid Chromatography

Zur Quantifizierung von D-Psicose, D-Fructose sowie der Zwischenstufe Allitol mittels HPLC (High Performance Liquid Chromatography) wurde ein Agilent HPLC 1260 Infinity II Series System verwendet. Die Detektion erfolgte mittels eines Brechungsindex-Detektors (RI-Detektion). Zur Messung wurde eine Phenomenex Rezex RPM-Monosaccharide Pb+2 (8%) Säule mit entsprechender Vorsäule verwendet und mit Reinstwasser isokratisch eluiert.

### High Performance Anion Exchange Chromatography

Zur Bestimmung der Substrat-Umsätze und Produkt-Konzentrationen bei der Oxidation von Allitol zu Allose wurde HPAEC verwendet. Die Analyten wurden mittels gepulster amperometrischer Detektion (PAD) detektiert. Dazu wurde eine Dionex^{™} CarboPac^{™} PA210-fast-4µm Säule mit entsprechender Vorsäule verwendet und mit einem NaOH-Gradienten eluiert.

### Bestimmung von Enzym-Aktivitäten (optisch-enzymatischer Assay)

Enzym-Aktivitäten in den Lysaten wurden mit einem Shimadzu UV-1900 Spektrophotometer bestimmt. Dazu wurde die Bildung bzw. der Verbrauch von NAD(P)H bei einer Wellenlänge von 340 nm über die Änderung der Absorption verfolgt. Die Messungen wurden mit 0,2 mM Kofaktor (NAD(P)⁺ oder NAD(P)H) durchgeführt. Dazu wurden 20 µl einer 10 mM Stock-Lösung des Kofaktors in einer Küvette (Greiner bio-one Semi-Micro-Küvette aus Polystyrol) vorgelegt und der gewünschte pH-Wert wurde mit 100 mM TEA-HCl-Puffer eingestellt (870 µl). 10 µl Lysat (verdünnt oder unverdünnt) und 100 µl Substrat-Lösung wurden zur Küvette zugesetzt und die Messung unmittelbar danach gestartet. Die Messungen wurden standardmäßig bei 25 °C durchgeführt. Über den Extinktionskoeffizienten von NADH bzw. NADPH bei 340 nm (*ε* = 6220 L mol⁻¹ cm⁻¹) kann die Enzym-Aktivität des Lysats in U/ml (bezogen auf das Volumen des Lysats) oder U/g (bezogen auf die zur Herstellung eingesetzte Biomasse) bestimmt werden. 1 U steht dabei für 1 µmol Substratumsatz pro Minute (1 U = 1 µmol/min = 1,67·10⁻⁸ kat).

Mit den nachfolgenden Beispielen werden bevorzugte Varianten des erfindungsgemäßen Verfahrens noch näher beschrieben. Die in diesen Beispielen eingesetzten Lysate wurden nach den oben beschriebenen Verfahren hergestellt.

### Beispiel 1

### Oxidation von Allitol zu Allose

In einem 2 ml Glas-Vial wurden folgende Komponenten vermischt: 155,7 µl deionisiertes Wasser, 125 µl eines 400 mM TEA-HCl-Puffers (pH 8), 125 µl einer Allitol-Lösung (200 g/l), 50 µl Reduktase-Lysat (Retsch), 10 U NAD(P)H-Oxidase-Lysat sowie 5 µl einer 5 mM NADP⁺-Lösung. Das Vial wurde in einem Eppendorf-Thermomixer unter kontinuierlichem Schütteln (30 °C, 800 rpm) für 20 h inkubiert.

Zur Analyse wurden 40 µl des Ansatzes mit 200 µl Methanol versetzt und im Eppendorf-Thermomixer bei 60 °C und 1200 rpm für 15 min inkubiert. Die Probe wurde in einer Zentrifuge kurz zentrifugiert, mit 760 µl Reinstwasser versetzt, gevortext und anschließend für 5 min bei max. g zentrifugiert. Der Überstand wurde 1:400 mit Reinstwasser verdünnt und mittels HPAEC (PAD) vermessen.

Auf diese Weise wurden 85,2% des Allitols (50 g/l) zu Allose umgesetzt.

### Beispiel 2

### Herstellung von Allose aus D-Fructose (Eintopf-Verfahren)

In einem 2 ml Glas-Vial wurden folgende Komponenten vermischt: 70,6 µl deionisiertes Wasser, 250 µl eines 200 mM TEA-HCl-Puffers (pH 8), 50 µl einer D-Fructose-Lösung (500 g/l) und 25 µl D-Psicose-3-Epimerase-Lysat. Danach wurden 35 µl Ribitol-Dehydrogenase-Lysat, 8 U Alkoholdehydrogenase-Lysat, 5 µl einer 10 mM NAD⁺-Lösung sowie 50 µl 2-Propanol zum Ansatz hinzugefügt. Der Ansatz wurde unter kontinuierlichem Schütteln (35 °C, 800 rpm) für insgesamt 20 h inkubiert.

Danach wurde der Ansatz 60 min lang auf 70 °C erhitzt. Nach Abkühlen wurden 25 µl Reduktase -Lysat (Sonifier), 10 U NAD(P)H-Oxidase-Lysat sowie 5 µl einer 5 mM NADP⁺-Lösung zugesetzt. Der Ansatz wurde in einem Eppendorf-Thermomixer unter kontinuierlichem Schütteln (24 °C, 800 rpm) für weitere 20 h inkubiert.

Zur Analyse wurden 100 µl des Ansatzes mit 200 µl Methanol versetzt und im Eppendorf Thermomixer bei 60 °C und 1200 rpm für 15 min inkubiert. Die Probe wurde in einer Zentrifuge kurz zentrifugiert, mit 700 µl deionisiertem Wasser versetzt, gevortext und anschließend für 5 min bei max. g zentrifugiert. 200 µl des Überstands wurden in ein HPLC-Vial mit Insert überführt und mittels HPLC (RI-Detektion) vermessen. Der Überstand wurde 1:1000 mit Reinstwasser verdünnt und mittels HPAEC (PAD) vermessen.

Auf diese Weise wurden 43,5% der D-Fructose (50 g/l) zu Allose umgesetzt, der Rest entfiel auf das Intermediat Allitol.

### Literatur

Kannan, R. R. R., Arumugam, R., & Anantharaman, P. (2012). Chemical composition and antibacterial activity of Indian seagrasses against urinary tract pathogens. Food Chemistry, 135(4), 2470-2473. https://doi.org/10.1016/j.foodchem.2012.07.070
Perold, G. W., Beylis, P., & Howard, A. S. (1973). Metabolites of proteaceae. Part VIII. The occurrence of (+)-D-allose in nature: rubropilosin and pilorubrosin from Protea rubropilosa beard. Journal of the Chemical Society, Perkin Transactions 1, 1973, 643-649. https://doi.org/10.1039/P19730000643
Mooradian, A. D., Smith, M., & Tokuda, M. (2017). The role of artificial and natural sweeteners in reducing the consumption of table sugar: a narrative review. Clinical Nutrition Espen, 18, 1-8. https://doi.org/10.1016/j.clnesp.2017.01.004
Tohi, Y., Taoka, R., Zhang, X., Matsuoka, Y., Yoshihara, A., Ibuki, E., Haba, R., Akimitsu, K., Izumori, K., Kakehi, Y., & Sugimuto, M. (2022). Antitumor Effects of Orally Administered Rare Sugar D-Allose in Bladder Cancer. International Journal of Molecular Sciences, 23(12), 6771. https://doi.org/10.3390/ijms23126771
Ishihara, Y., Katayama, K., Sakabe, M., Kitamura, M., Aizawa, M., Takara, M., & Itoh, K. (2011). Antioxidant properties of rare sugar D-allose: Effects on mitochondrial reactive oxygen species production in Neuro2A cells. Journal of Bioscience and Bioengineering, 112(6), 638-642. https://doi.org/10.1016/j.jbiosc.2011.08.005
Gao, D., Kawai, N., & Tamiya, T. (2011). The anti-inflammatory effects of D-allose contribute to attenuation of cerebral ischemia-reperfusion injury. Medical Hypotheses, 76(6), 911-913. https://doi.org/10.1016/j.mehy.2011.03.007
Chen, Z., Chen, J., Zhang, W., Zhang, T., Guang, C., & Mu, W. (2018). Recent research on the physiological functions, applications, and biotechnological production of D-allose. Applied Microbiology and Biotechnology, 102, 4269-4278. https://doi.org/10.1007/s00253-018-8916-6
Lim, Y.-R., & Oh, D.-K. (2011). Microbial metabolism and biotechnological production of D-allose. Applied Microbiology and Biotechnology, 91, 229-235. https://doi.org/10.1007/s00253-011-3370-8
Mijailovic, N., Nesler, A., Perazzolli, M., Aït Barka, E., & Aziz, A. (2021). Rare Sugars: Recent Advances and Their Potential Role in Sustainable Crop Protection. Molecules, 26(6), 1720. https://doi.org/10.3390/molecules26061720
Jumde, V. R., Eisink, N. N. H. M., Witte, M. D., & Minnaard, A. J. (2016). C3 Epimerization of Glucose, via Regioselective Oxidation and Reduction. The Journal of Organic Chemistry, 81(22), 11439-11443. https://doi.org/10.1021/acs.joc.6b02074
Nam, K. H. (2022). Glucose Isomerase: Functions, Structures, and Applications. Applied Sciences, 12(1), 428. https://doi.org/10.3390/app12010428
Zhang, W., Yu, S., Zhang, T., Jiang, B., & Mu, W. (2016). Recent advances in D-allulose: Physiological functionalities, applications, and biological production. Trends in Food Science and Technology, 54, 127-137. https://doi.org/10.1016/j.tifs.2016.06.004
Jiang, S., Xiao, W., Zhu, X., Yang, P., Zheng, Z., Lu, S., Jiang, S., Zhang, G., & Liu, J. (2020). Review on D-Allulose: In vivo Metabolism, Catalytic Mechanism, Engineering Strain Construction, Bio-Production Technology. Frontiers in Bioengineering and Biotechnology, 8, 26. https://doi.org/10.3389/fbioe.2020.00026
Lee, T. E., Shin, K. C., & Oh, D. K. (2018). Biotransformation of Fructose to Allose by a One-Pot Reaction Using Flavonifractor plautii D-Allulose 3-Epimerase and Clostridium thermocellum Ribose 5-Phosphate Isomerase. Journal of Microbiology and Biotechnology, 28(3), 418-424. https://doi.org/10.4014/jmb.1709.09044
Li, C., Gao, L., Du, K., Lin, H., Ren, Y., Lin, J., & Lin, J. (2020). Production of D-allose from D-fructose using immobilized L-rhamnose isomerase and D-psicose 3-epimerase. Bioprocess and Biosystems Engineering, 43, 645-653. https://doi.org/10.1007/s00449-019-02262-y
Choi, M. N., Shin, K.-C., Kim, D. W., Kim, B.-J., Park, C.-S., Yeom, S.-J., Kim, Y.-S. (2021). Production of D-Allose From D-Allulose Using Commercial Immobilized Glucose Isomerase. Frontiers in Bioengineering and Biotechnology, 9, 681253. https://doi.org/10.3389/fbioe.2021.681253
Menavuvu, B. T., Poonperm, W., Leang, K., Noguchi, N., Okada, H., Morimoto, K., Granström, T. B., Takada, G., & Izumori, K. (2006). Efficient biosynthesis of D-allose from D-psicose by cross-linked recombinant L-rhamnose isomerase: Separation of product by ethanol crystallization. Journal of Bioscience and Bioengineering, 101(4), 340-345. https://doi.org/10.1263/jbb.101.340
Morimoto, K., Park C.-S., Ozaki, M., Takeshita, K., Shimonishi, T., Granström, T. B., Takata, G., Tokuda, M., & Izumori, K. (2006). Large scale production of D-allose from D-psicose using continuous bioreactor and separation system. Enzyme and Microbial Technology, 38(6), 855-859. https://doi.org/10.1016/j.enzmictec.2005.08.014
Zheng, L.-J., Guo, Q., Zhang, Y.-X., Liu, C.-Y., Fan L.-H., & Zheng, H.-D. (2022). Engineering of Escherichia coli for D-allose fermentative synthesis from D-glucose through izumoring cascade epimerization. Frontiers in Bioengineering and Biotechnology, 10, 1050808. https://doi.org/10.3389/fbioe.2022.1050808
Li, Y., Shi, T., Han, P., & You, C. (2021). Thermodynamics-Driven Production of Value-Added D-Allulose from Inexpensive Starch by an In Vitro Enzymatic Synthetic Biosystem. ACS Catalysis, 11(9), 5088-5099. https://doi.org/10.1021/acscatal.0c05718
Terami, Y., Uechi, K., Nomura, S., Okamoto, N., Morimoto, K., & Takata, G. (2015). Production of L-allose and D-talose from L-psicose and D-tagatose by L-ribose isomerase. Bioscience, Biotechnology, and Biochemistry, 79(10), 1725-1729. https://doi.org/10.1080/09168451.2015.1038215
Izumori, K. (2006). Izumoring: a strategy for bioproduction of all hexoses. Journal of Biotechnology, 124(4), 717-722. https://doi.org/10.1016/j.jbiotec.2006.04.016
Hassanin, H. A. M., Mu, W., Koko, M. Y. F., Zhang, T., Masamba, K., & Jiang, B. (2017). Allitol: production, properties and applications. International Journal of Food Science and Technology, 52(1), 91-97. https://doi.org/10.1111/ijfs.13290
Takeshita, K., Ishida, Y., Takada, G., & Izumori, K. (2000). Direct Production of Allitol from D-Fructose by a Coupling Reaction Using D-Tagatose 3-Epimerase, Ribitol Dehydrogenase and Formate Dehydrogenase. Journal of Bioscience and Bioengineering, 90(5), 545-548. https://doi.org/10.1016/51389-1723(01)80038-4
Wang, L., Chen, K., Zheng, P., Huo, X., Liao, F., Zhu, L., Hu, M., & Tao, Y. (2023). Enhanced production of D-psicose from D-fructose by a redox-driven multi-enzyme cascade system. Enzyme and Microbial Technology, 163, 110172. https://doi.org/10.1016/j.enzmictec.2022.110172
Lin, B., & Tao, Y. (2017). Whole-cell biocatalysts by design. Microbial Cell Factories, 16, 106. https://doi.org/10.1186/s12934-017-0724-7
Altschul, S. F., Gish, W., Miller, W., Myers, E. W., & Lipman, D. J. (1990). Basic local alignment search tool. Journal of Molecular Biology, 215(3), 403-410. https://doi.org/10.1016/S0022-2836(05)80360-2
Henikoff, S., & Henikoff, J. G. (1992). Amino acid substitution matrices from protein blocks. Proceedings of the National Academy of Sciences of the United States of America, 89(22), 10915-10919. https://doi.org/10.1073/pnas.89.22.10915
Sambrook, J., Fritsch, E. R., & Maniatis, T. (1989). Molecular Cloning: A Laboratory Manual (2nd ed.). Cold Spring Harbor, NY: Cold Spring Harbor Laboratory Press.
Mu, W., Chu, F., Xing, Q., Yu, S., Zhou, L., & Jiang, B. (2011). Cloning, Expression, and Characterization of a D-Psicose 3-Epimerase from Clostridium cellulolyticum H10. Journal of Agricultural and Food Chemistry, 59(14), 7785-7792. https://doi.org/10.1021/jf201356q
Chan, H.-C., Zhu, Y., Hu, Y., Ko, T.-P., Huang, C.-H., Ren, F., Chen, C.-C., Ma, Y., Guo, R.-T., & Sun, Y. (2012). Crystal structures of D-psicose 3-epimerase from Clostridium cellulolyticum H10 and its complex with ketohexose sugars. Protein & Cell, 3(2), 123-131. https://doi.org/10.1007/s13238-012-2026-5
Protein [Internet]. Bethesda (MD): National Library of Medicine (US), National Center for Biotechnology Information. Accession No. WP_265716617.1, SDR family oxidoreductase [Klebsiella pneumoniae]; [aufgerufen am 06.07.2023]. https://www.ncbi.nlm.nih.gov/protein/WP_265716617.1
Sakoda, H., & Imanaka, T. (1992). Cloning and sequencing of the gene coding for alcohol dehydrogenase of Bacillus stearothermophilus and rational shift of the optimum pH. Journal of Bacteriology, 174(4), 1397-1402. https://doi.org/10.1128/jb.174.4.1397-1402.1992
Petschacher, B., Staunig, N., Müller, M., Schürrmann, M., Mink, D., De Wildeman, S., Gruber, K., & Glieder, A. (2014). COFACTOR SPECIFICITY ENGINEERING OF STREPTOCOCCUS MUTANS NADH OXIDASE 2 FOR NAD(P)+ REGENERATION IN BIOCATALYTIC OXIDATIONS. Computational and Structural Biotechnology Journal, 9(14), e201402005. https://doi.org/10.5936/csbj.201402005

## Patentansprüche

1. Verfahren zur Herstellung einer wässerigen Lösung enthaltend Allose, indem aus D-Fructose, welche in einer wässerigen Lösung gelöst vorliegt, durch Behandeln mit einer Epimerase *in vitro* D-Psicose gebildet wird, wonach die D-Psicose durch Behandeln mit einer NAD(P)H-abhängigen Oxidoreduktase *in vitro* zu Allitol reduziert wird, wonach die Epimerase und die NAD(P)H-abhängige Oxidoreduktase deaktiviert oder durch Ultrafiltration entfernt werden und anschließend das Allitol enzymatisch *in vitro* zu Allose oxidiert wird.

2. Verfahren nach Anspruch 1, **dadurch gekennzeichnet, dass** der durch die Reduktion entstandene oxidierte Kofaktor NAD(P)⁺ mittels einer Alkoholdehydrogenase und einem sekundären Alkohol unter Bildung eines Ketons reduziert wird.

3. Verfahren nach Anspruch 2, **dadurch gekennzeichnet, dass** der sekundäre Alkohol 2-Propanol ist.

4. Verfahren nach einem der Ansprüche 1 bis 3, **dadurch gekennzeichnet, dass** die enzymatische Oxidation des Allitols zu Allose mit einer entsprechenden NAD(P)⁺-abhängigen Oxidoreduktase durchgeführt wird.

5. Verfahren nach einem der Ansprüche 1 bis 3, **dadurch gekennzeichnet, dass** es als Eintopf-Reaktion ohne Isolierung von Zwischenprodukten durchgeführt wird.

6. Verfahren zur Herstellung einer wässerigen Lösung enthaltend Allose, indem Allitol oxidiert wird, **dadurch gekennzeichnet, dass** die Oxidation enzymatisch *in vitro* durchgeführt wird.

7. Verfahren nach Anspruch 6, **dadurch gekennzeichnet, dass** die enzymatische Oxidation des Allitols zu Allose mit einer entsprechenden NAD(P)⁺-abhängigen Oxidoreduktase durchgeführt wird.

8. Verfahren nach einem der Ansprüche 1 bis 7, **dadurch gekennzeichnet, dass** die Enzyme als Lysat der entsprechenden, sie bildenden Zellen vorliegen.

9. Verfahren nach einem der Ansprüche 1 bis 8, **dadurch gekennzeichnet, dass** der durch die Oxidation entstandene, reduzierte Kofaktor NAD(P)H mittels einer Oxidase und Sauerstoff zu NAD(P)⁺ oxidiert wird.

10. Verfahren nach einem der Ansprüche 1 bis 9, **dadurch gekennzeichnet, dass** die enzymatische Oxidation des Allitols zu Allose mit einer NAD(P)⁺-abhängigen Oxidoreduktase durchgeführt wird, die eine Aminosäuresequenz umfasst, welche ausgewählt ist aus der Gruppe bestehend aus:
i) einer Aminosäuresequenz, die eine Identität zu SEQ ID Nr. 1, SEQ ID Nr. 3, SEQ ID Nr. 5, SEQ ID Nr. 7, SEQ ID Nr. 9, SEQ ID Nr. 11, SEQ ID Nr. 13, SEQ ID Nr. 15 oder SEQ ID Nr. 17 von mindestens 75% aufweist,
ii) einer Aminosäuresequenz, die durch eine Nukleinsäure kodiert wird, die eine Identität zu SEQ ID Nr. 2, SEQ ID Nr. 4, SEQ ID Nr. 6, SEQ ID Nr. 8, SEQ ID Nr. 10, SEQ ID Nr. 12, SEQ ID Nr. 14, SEQ ID Nr. 16 oder SEQ ID Nr. 18 von mindestens 75% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 2, SEQ ID Nr. 4, SEQ ID Nr. 6, SEQ ID Nr. 8, SEQ ID Nr. 10, SEQ ID Nr. 12, SEQ ID Nr. 14, SEQ ID Nr. 16 oder SEQ ID Nr. 18 bindet.

11. Verwendung einer NAD(P)⁺-abhängigen Oxidoreduktase, die eine Aminosäuresequenz umfasst, welche ausgewählt ist aus der Gruppe bestehend aus:
i) einer Aminosäuresequenz, die eine Identität zu SEQ ID Nr. 1, SEQ ID Nr. 3, SEQ ID Nr. 5, SEQ ID Nr. 7, SEQ ID Nr. 9, SEQ ID Nr. 11, SEQ ID Nr. 13, SEQ ID Nr. 15 oder SEQ ID Nr. 17 von mindestens 75% aufweist,
ii) einer Aminosäuresequenz, die durch eine Nukleinsäure kodiert wird, die eine Identität zu SEQ ID Nr. 2, SEQ ID Nr. 4, SEQ ID Nr. 6, SEQ ID Nr. 8, SEQ ID Nr. 10, SEQ ID Nr. 12, SEQ ID Nr. 14, SEQ ID Nr. 16 oder SEQ ID Nr. 18 von mindestens 75% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 2, SEQ ID Nr. 4, SEQ ID Nr. 6, SEQ ID Nr. 8, SEQ ID Nr. 10, SEQ ID Nr. 12, SEQ ID Nr. 14, SEQ ID Nr. 16 oder SEQ ID Nr. 18 bindet,
zur Oxidation von Allitol zu Allose.
